# EUROPEAN PATENT APPLICATION

(11) **EP 2 484 362 A2**
(43) Date of publication of application: **08.08.2012**
(21) Application number: 10819941.5
(22) Date of filing: 02.09.2010
(51) Int. Cl.: A61K 31/4353

(54) **USE OF GYMNODIMINE, ANALOGUES AND DERVIATIVES FOR THE TREATMENT AND/OR PREVENTION OF NEURODEGENERATIVE DISEASES ASSOCIATED WITH TAU AND B-AMYLOID**

(30) Priority: 30.09.2009 ES 200930763
(71) Applicant: Universidade De Santiago De Compostela, A Coruña (ES)
(72) Inventor: BOTANA LÓPEZ, Luis Miguel, E-15782 Santiago de Compostela (A Coruña) (ES); ALONSO LÓPEZ, Eva, E-15782 Santiago de Compostela (A Coruña) (ES); VALE GONZÁLEZ, Carmen, E-15782 Santiago de Compostela (A Coruña) (ES)
(74) Representative: Pons Ariño, Angel
(86) International application number: PCT/ES2010/070578
(87) International publication number: WO 2011/039394

(57) **Abstract**

The present invention refers to the use of a compound with the chemical structure 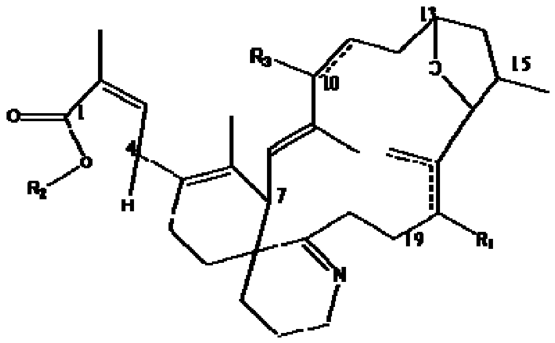 for the preparation of a medicinal drug, preferably for the prevention or the treatment of pathological processes related to the tau and β-amyloid proteins. The compound preferably used is gymnodimine.

## Description

The present invention is in the field of biomedicine. Specifically, it refers to the use of a compound with the chemical structure: 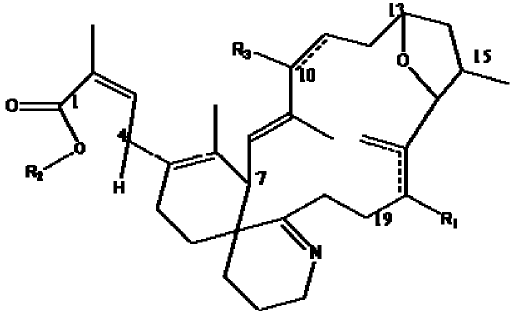 for the preparation of a medicinal drug, preferably for the prevention or the treatment of pathological processes related to the tau and β-amyloid proteins, such as for example Alzheimer's disease.

### STATE OF PRIOR ART

Alzheimer's disease is a progressive neurodegenerative disease, of unknown origin, and for which no preventative or curative treatment can currently be offered. This disease affects between 5 % and 7 % of people over sixty-five years of age and is currently the most common cause of invalidity and dependence in elderly people. It is estimated that 8 million Europeans are affected by Alzheimer's disease and, taking into account the aging of the population, it is predicted that the number of sufferers will double by 2020 and triple by 2050.

This disease is characterised by a progressive loss of memory and other mental capacities as the neurones degenerate and different areas of the brain atrophy. At the neuropathological level, Alzheimer's disease is characterised by the appearance of two abnormal structures that accumulate in the brain. These structures are amyloid deposits or plaques and neurofibrillary tangles.

The amyloid deposits are insoluble fibres located intra- and extracellularly, formed by the β-amyloid (βA) peptide, more specifically by the βA40 and βA42 forms, which are generated by the sequential proteolytic rupture of the β-amyloid precursor protein (APP) by β-secretases and γ-secretases (Shirwany et al. 2007 Neuropsychiatric Disease and Treatment; 3, 597-612). This peptide is found in the normal form in the brain in pico or nanomolar amounts. In these amounts, the peptide is in a soluble form. When an increase in β-amyloid occurs by anomalous processing of the β-amyloid precursor protein (APP), this becomes insoluble, giving rise to the formation of deposits. Different mutations in the β-amyloid precursor protein (APP) are related to Alzheimer's disease due to an increase or abnormality in the transformation of APP into β-amyloid. In patients with Alzheimer's disease, β-amyloid aggregates appear in specific cerebral regions, inducing an inflammatory response, neuronal death and progressive cognitive deterioration. This β-amyloid peptide has also been involved in neuropathological defects in individuals with Down's syndrome.

The neurofibrillary tangles by contrast are intracellular filaments formed by the polymerisation of the tau protein, which normally acts as a protein associated with microtubules of neurone axons. These structures, which accumulate in the cytoplasm of degenerated neurones, were called "paired helical filaments or PHFs. These show characteristics that are different from those of normal neurofilaments and microtubules. The main constituent of the PHFs is phosphorylated tau protein. Hyperphosphorylation of tau is due either to an increase in tau expression because there is a higher quantity of substrate that can be phosphorylated or to hyperphosphorylation by kinases. This abnormal protein phosphorylation of tau is intimately related to the abnormal aggregation of the protein. Tau hyperphosphorylation is currently involved in about 22 pathologies, including Alzheimer's disease, frontal lobe dementia (also called frontotemporal neurodegeneration), corticobasal degeneration, Pick's disease and Parkinson's disease with dementia.

Initially, studies were undertaken to try to independently elucidate the involvement of both tau and β-amyloid in Alzheimer's disease. The first attempts at treatment of the disease were also directed at improving the effects of each of these proteins independently. Currently, studies carried out have shown that both proteins may be related because amyloid deposits may affect different molecular pathways that facilitate tau phosphorylation and its subsequent aggregation (Blurton-Jones et al. 2006, Current Alzheimer Research, 3(5), 435-448). Amyloid deposits may also activate various specific kinases that increase hyperphosphorylation of the tau protein and therefore the formation of neurofibrillary tangles. Despite this relation, other studies carried out indicate that improvement in the abnormality in one of the proteins is not necessarily linked to the improvement in the other, leading in some cases to an increase in abnormality (Oddo et al., 2005. Proc Natl Acad Sci U.S.A, 102(8), 3046-51). Therefore it is necessary to carry out studies in models presenting both pathologies simultaneously.

There are currently various treatments for Alzheimer's disease that do not allow curing of the disease but act to delay its progression. The only pharmaceutical or medicinal drug approved for the treatment of the disease when it has already advanced to a moderate or severe level, that is in an advanced state, is memantine, a non-competitive antagonist of NMDA (N-methyl-D-aspartate) receptors, which prevents the toxic effect of high glutamate concentrations in neurones. Other compounds, in this case used to prevent the development of the disease, are, for example, donepezil or rivastigmine, which act by inhibiting acetylcholinesterase, increasing the levels of the neurotransmitter acetylcholine (A. Fisher 2008, Neurotherapeutics; 5:433-442).

All this suggests that future studies of Alzheimer's disease and other neurodegenerative diseases will be directed towards the search of medicinal drugs acting on both disorders and therefore leading to complete improvement of the disease.

Currently, one of the most important sources of compounds that may be useful for the production of pharmaceuticals is the marine environment. Here a multitude of biochemical resources have been found and have been demonstrated to have great use in healthcare such as, for example, pharmaceuticals with anti-tumour activity. Included in these compounds, marine phycotoxins can have extensive clinical application because of their high diversity and, therefore, multiple cellular mechanisms of action and induced responses.

A recently discovered marine phycotoxin is gymnodimine. It was described for the first time in 1994 and it was initially believed to be a compound produced by the dinoflagellate *Gymnodinium*. Later it was discovered that the toxin was produced by a complex formed by two species of dinoflagellates, which currently have been renamed as *Karenia selliformis*.

The phycotoxin has a spirocyclic imine ring structure and is included in the cyclic imine class. The main pharmacological target is the central nervous system, as its toxicity is much higher when it is introduced intracerebrally than intraperitoneally. The compound exhibits significantly reduced toxicity when orally administered (Munday et al. 2004. Toxicon, 44: 173-178). Gymnodimine exercises its toxic effect via high affinity binding to the nicotinic acetylcholine receptors, where it has a blocking effect (Kharrat et al. 2008. Journal of Neurochemistry; 107: 952-963; Dragunow et al. 2005, Environmental Toxicology and Pharmacology; 20: 305-312).

Therefore, there is a need to find an effective treatment that acts on the two major elements involved in the progression of diseases such as Alzheimer's, which are the β-amyloid deposits and tau hyperphosphorylation.

### DESCRIPTION OF THE INVENTION

The present invention refers to the use of a compound with the chemical structure (I): 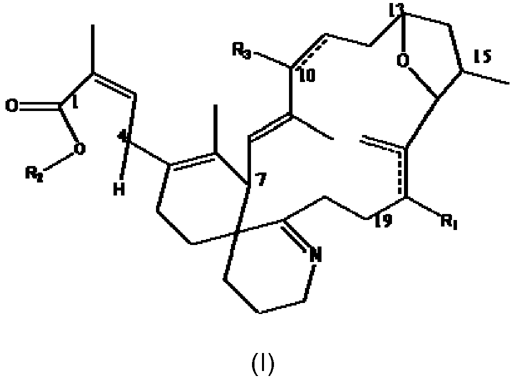 for the preparation of a medicament, preferably for the prevention and/or treatment of pathological processes related to the tau and/or β-amyloid proteins, such as for example Alzheimer's disease.

In the examples of the present invention, to see the effect of gymnodimine on the overexpression of beta-amyloid and on hyperphosphorylation of tau, in vitro cultures of cortical neurones were used, obtained from triple-transgenic mice, which simultaneously overexpressed the human transgenes for presenilin (PS1_{M146V}), β-amyloid precursor protein (APP_{Swe}) and tau protein (taU_{P301L}). Simultaneous overexpression of these 3 elements is related to accumulation of β-amyloid and the formation of neurofibrillary plaques and therefore these cells are useful in the study of the efficacy of compounds against diseases related to increases in tau and β-amyloid. The examples of the present invention demonstrate that treatment with gymnodimine causes a reduction of β-amyloid levels and tau phosphorylation, at both the Ser202 site and the Thr212 and Ser214 sites.

It should be pointed out that the present invention shows that gymnodimine, although it is described as a toxin, does not cause a reduction in cell viability at a maximum concentration of 100 nM in the neuronal model used for this study.

Gymnodimine is a compound belonging to the cyclic imine group and has the chemical structure (II). The formula is C₃₂H₄₅NO₄, and its molecular weight is approximately 507 Daltons. It is a toxin of marine origin, produced by a complex of two species of dinoflagellates called *Karenia selliformis.*

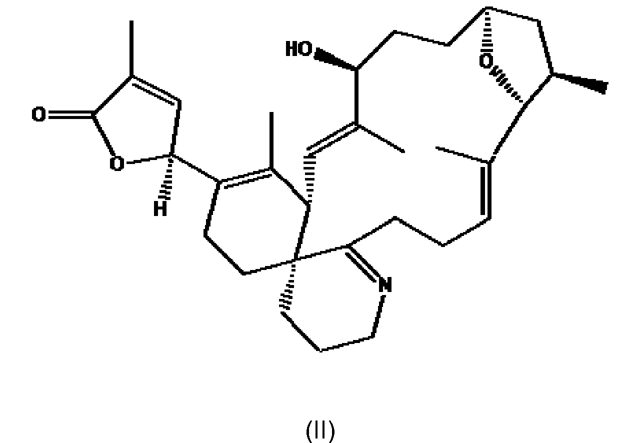

The activation of nicotinic receptors by the administration of nicotine has been described to help in the protection against the disease. Surprisingly, gymnodimine binds with high affinity to the nicotinic acetylcholine receptors, blocking them.

Two analogues of gymnodimine have been described called gymnodimine B (chemical structure III) and gymnodimine C (chemical structure IV) with the formula C₃₂H₄₅NO₅, which have a chemical structure similar to gymnodimine and differ in their chirality.

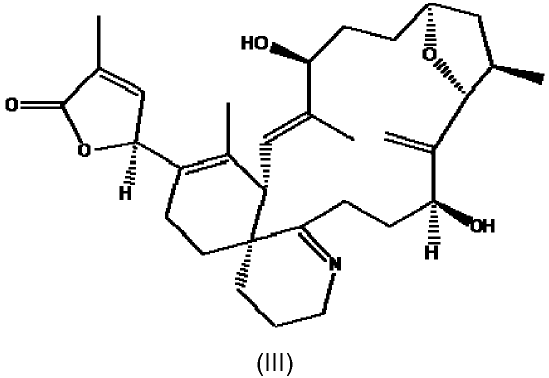

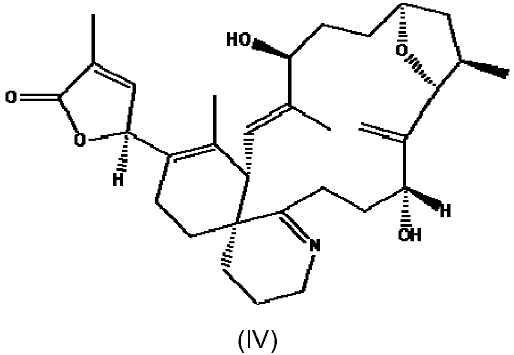

This molecule can be subjected to modifications giving rise to various derivatives that may have similar functionality. All these compounds, both analogues and derivatives, have a common chemical structure (I) to that of gymnodimine, in which - - - - - represents a possible double bond, R₁ can be a hydrogen or hydroxyl (OH) or alkoxyl (OR) group, R₂ is a hydrogen or does not exist and the oxygen binds to carbon 4 making a furanone ring, and R₃ is a hydrogen, a hydroxyl (OH) or an alkoxyl (OR) group.

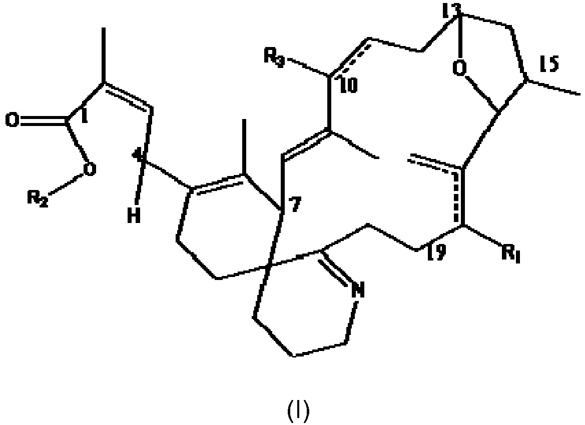

Therefore, a first aspect of the invention refers to the use of a compound of the chemical structure (I) where:
- - - - - represents a possible double bond,
R₁ can be hydrogen, a hydroxyl (OH) or an alkoxyl (OR) group,
R₂ is a hydrogen or does not exist and the oxygen binds to carbon 4 giving rise to a furanone ring, and
R₃ is hydrogen, a hydroxyl (OH) or an alkoxyl (OR) group,
for the preparation of a medicinal drug.

In the case of gymnodimine there is a double bond between carbon atoms 17 and 18, R₁ is hydrogen, R₂ does not exist so the oxygen is bound to carbon 4 giving a furanone ring and R₃ is a hydroxyl (OH) group. Therefore, a preferred embodiment of this aspect of the invention refers to the use of a compound of the chemical structure (I) where:
there is a double bond between carbon atoms 17 and 18,
R₁ is hydrogen,
R₂ does not exist so the oxygen is bound to carbon 4 making a furanone ring, and
R₃ is a hydroxyl (OH) group,
for the preparation of a medicament.

The term "analogue" as used here refers to a chemical substance similar to another chemical substance in structure and / or function. For example, analogues of gymnodimine can be considered, without limitation, to be gymnodimine B and gymnodimine C.

The present invention considers the term "derivative" to mean a compound that is produced starting from another by means of modifications made to the first and which has a similar functionality. Such modifications can be made, without limitation, by chemical, physical, microbiological of pharmacological methods.

Increase in β-amyloid and phosphorylation of tau, either separately or together, is often associated with a pathological process. These processes are fundamentally related to the nervous system because accumulation basically takes place in neurones, causing their degradation. The main pathology related to the two features occurring together is Alzheimer's disease. This disease progresses with an increase in β-amyloid deposits and tau hyperphosphorylation, giving rise to neurofibrillary tangles that induce progressive degeneration of neurones and therefore cognitive and motor deterioration. As demonstrated in the examples, gymnodimine is capable of reducing overexpression of β-amyloid and hyperphosphorylation of tau, but does not have any effects on these structures unless they are altered. This indicates that these compounds are useful for the treatment of pathologies related to the increase in β-amyloid expression or tau hyperphosphorylation both independently and together. Therefore, a preferred embodiment of this aspect of the invention refers to the use of a compound of chemical structure (I) for the preparation of a medicinal drug for the prevention and/or treatment of a pathology related to increase in β-amyloid and/or hyperphosphorylation of tau. A more preferred embodiment refers to the use of the compound of chemical structure (II) for the preparation of a medicinal drug for the prevention and/or treatment of a pathology related to increase in β-amyloid and/or hyperphosphorylation of tau.

"Pathology related to the increase of β-amyloid" in the present invention is considered to mean any pathology featuring, either an increase in the levels of the β-amyloid precursor protein or an increase in the anomalous processing of this protein, increasing the insoluble amount and therefore giving rise in both size and quantity of intra- and extra-cellular β-amyloid deposits. Included in these pathologies, for example but without limitation, are amyotrophic lateral sclerosis, Down's syndrome, vascular dementia, cerebral amyloid angiopathy related with prion proteins and Creutzfeldt-Jacob disease. Therefore, a preferred embodiment of this aspect of the invention refers to the use of a compound of chemical structure (I) for the preparation of a medicinal drug for the prevention and / or treatment of a pathology related to increase in β-amyloid that is selected from the list comprising: amyotrophic lateral sclerosis, Down's syndrome, vascular dementia, cerebral amyloid angiopathy related to prion proteins and Creutzfeld-Jacob's disease. A more preferred embodiment refers to the use of the compound of chemical structure (II) for the preparation of a medicinal drug for the prevention and/or treatment of a pathology related to increase in β-amyloid that is selected from the list comprising: amyotrophic lateral sclerosis, Down's syndrome, vascular dementia, cerebral amyloid angiopathy related to prion proteins and Creutzfeld-Jacob's disease.

"Pathology related to hyperphosphorylation of tau" in the present invention is considered to be any pathology featuring an increase in the expression of tau, which leads to an increase in the quantity of phosphorylated protein or a hyperphosphorylation of this protein, even without change in expression, as both situations lead to an increase in the size or number of neurofibrillary tangles caused by the anomalous aggregation of phosphorylated tau. Included in the pathologies related to tau hyperphosphorylation are, for example but without limitation, frontotemporal dementia, progressive supranuclear paralysis, dementia associated with multiple system tauopathy, corticobasal degeneration and frontotemporal lobular degeneration or Picks's disease. Therefore, another preferred embodiment of this aspect of the invention refers to the use of a compound of chemical structure (I) for the preparation of a medicinal drug for the prevention and/or treatment of a pathology related to hyperphosphorylation of tau that is selected from the list comprising: frontotemporal dementia, progressive supranuclear paralysis, dementia associated with multiple system tauopathy, corticobasal degeneration and frontotemporal lobular degeneration or Picks's disease. A more preferred embodiment of this aspect of the invention refers to the use of a compound of chemical structure (II) for the preparation of a medicinal drug for the prevention and/or treatment of a pathology related to hyperphosphorylation of tau that is selected from the list comprising: frontotemporal dementia, progressive supranuclear paralysis, dementia associated with multiple system tauopathy, corticobasal degeneration and frontotemporal lobular degeneration or Picks's disease.

There are many other diseases in addition to Alzheimer's that progress with simultaneous changes in both proteins such as, for example but without limitation, moderate cognitive disorders or deficits, hereditary cerebral hemorrhage with amyloidosis-Dutch type, cerebral amyloid angiopathy, dementia associated with Parkinson's disease, neurodegenerative disease due to diffuse Lewy bodies, corticobasal degeneration, sub-acute sclerosing panencephalitis, dementia with argyrophilic grain disease and familial Gerstmann-Straussler-Scheinker disease. Therefore, another preferred embodiment of this aspect of the invention refers to the use of a compound of chemical structure (I) for the preparation of a medicinal drug for the prevention and / or treatment of a pathology related to increase in β-amyloid and hyperphosphorylation of tau that is selected from the list comprising: Alzheimer's disease, moderate cognitive disorders or deficits, hereditary cerebral hemorrhage with amyloidosis-Dutch type, cerebral amyloid angiopathy, dementia associated with Parkinson's disease, neurodegenerative disease due to diffuse Lewy bodies, corticobasal degeneration, sub-acute sclerosing panencephalitis, dementia with argyrophilic grain disease and familial Gerstmann-Straussler-Scheinker disease. A more preferred embodiment of this aspect of the invention refers to the use of a compound of chemical structure (II) for the preparation of a medicinal drug for the prevention and / or treatment of a pathology related to β-amyloid increase and tau hyperphosphorylation that is selected from the list comprising: Alzheimer's disease, moderate cognitive disorders or deficits, hereditary cerebral hemorrhage with amyloidosis-Dutch type, cerebral amyloid angiopathy, dementia associated with Parkinson's disease, neurodegenerative disease due to diffuse Lewy bodies, corticobasal degeneration, sub-acute sclerosing panencephalitis, dementia with argyrophilic grain disease and familial Gerstmann-Straussler-Scheinker disease. "Moderate cognitive disorders or deficits" in the present invention is considered to mean those changes of a person's intellectual faculties that include, without limitation, deterioration of orientation, deterioration of short term memory, deterioration of reasoning, problems with calculation, problems with language, change in the ability to carry out complex tasks and change in programming ability that appear in the initial states of different diseases such as, for example but without limitation, Alzheimer's disease, schizophrenia or senile dementia.

Another aspect of the invention refers to a pharmaceutical composition, (hereafter the pharmaceutical composition of the invention) that comprises a compound of chemical structure (I) or chemical structure (II). In a preferred embodiment, the pharmaceutical composition also comprises a pharmaceutically acceptable vehicle. In another preferred embodiment, the pharmaceutical composition also comprises another active ingredient. In a more preferred embodiment of this aspect of the invention, the pharmaceutical composition comprises a compound of chemical structure (I) or chemical structure (II) and also another active ingredient with a pharmaceutically acceptable vehicle.

The composition of the present invention can be formulated for administration to an animal, and more preferably a mammal, including man, in a variety of forms known in the state of the art. Therefore, it can be administered, without limitation, in aqueous or non-aqueous solutions, in emulsions or in suspensions. Examples of non-aqueous solutions are, for example but without limitation, propylene glycol, polyethylene glycol, vegetable oils such as olive oil, or injectable organic esters such as ethyl oleate. Examples of aqueous solutions are, for example but without limitation, water, alcohol and water solutions or saline media. Aqueous solutions may be buffered or not, and can have additional active or inactive components. Additional components may include salts to modulate the ionic force, preservatives including, but without limitation, antimicrobial agents, antioxidants, chelating agents or similar, or nutrients including glucose, dextrose, vitamins or minerals. Alternatively, the compositions may be prepared for administration in solid form. Compositions may combine various inert vehicles or excipients including, without limitation: agglutinants such as microcrystalline cellulose, tragacanth gum or gelatine; excipients such as starch or lactose; dispersing agents such as alginic acid or corn starch; lubricants such as magnesium stearate; non-stick agents such as colloidal silicon dioxide; sweeteners such as sucrose or saccharine; or flavouring agents such as mint or methyl salicylate.

Such compositions and / or their formulation can be administered to an animal, including a mammal and, therefore, to man, in a variety of routes including, but without limitation, parenteral, intraperitoneal, intravenous, intradermal, intralesional, intra-arterial, intracardial, intramuscular, intranasal, intracraneal, subcutaneous, intraorbital, intracapsular, topical, by transdermal patches, via rectal, via vaginal or urethral, by administration of a suppository, percutaneous, nasal spray, surgical implant, internal surgical paint, infusion pump or via catheter.

The dose for obtaining a therapeutically effective amount depends on a variety of factors such as, for example, age, weight, gender or tolerance of the mammal. In the sense used in this description, the expression "therapeutically effective amount" refers to the amount of pharmaceutical composition of the invention that produces the desired effect and, in general, is determined by many factors including the properties of the pharmaceutical composition and the therapeutic effect desired.

### DESCRIPTION OF THE FIGURES

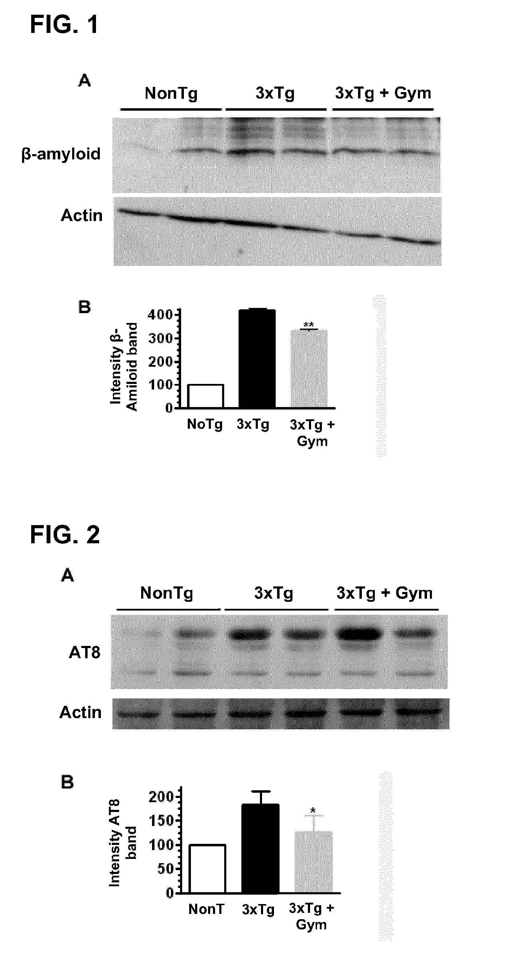

**Figure 1** shows the reduction in expression levels of intracellular β-amyloid after treatment with gymnodimine. (A) Western blot bands showing the expression of β-amyloid in neocortical cultures of wild animals (NonTg), neocortical cultures obtained from 3xTg-AD (3xTg) mice and levels of β-amyloid peptide in neocortical cultures of 3xTg-AD mice treated with gymnodimine (3xTg + Gym), evaluated with the 6E10 antibody. Exposure of triple-transgenic mouse cortical cultures to gymnodimine reduces overexpression of β-amyloid in this *in vitro* model. (B) Quantification of Western blot bands showing a significant reduction of overexpression of β-amyloid after treatment with gymnodimine (**p < 0.005 compared to expression of β-amyloid in transgenic cultures, n = 3, obtained in three representative experiments, each carried out in duplicate).
**Figure 2** shows a reduction in the levels of phosphorylated tau in cultures of transgenic neurones treated with gymnodimine. (A) Western blot images showing levels of tau phosphorylation using the AT8 antibody (recognises phosphorylated tau on Ser202) in wild cultures (NonTg), transgenic cultures (3xTg) and transgenic cultures treated with gymnodimine (3xTg Gym). Data obtained in a representative experiment. (B) Quantification of the expression of phosphorylated tau (marked with antibody AT8) showing a significant reduction of tau phosphorylation in transgenic cultures treated with gymnodimine (*p < 0.05, n = 3 obtained in three representative experiments, each carried out in duplicate).

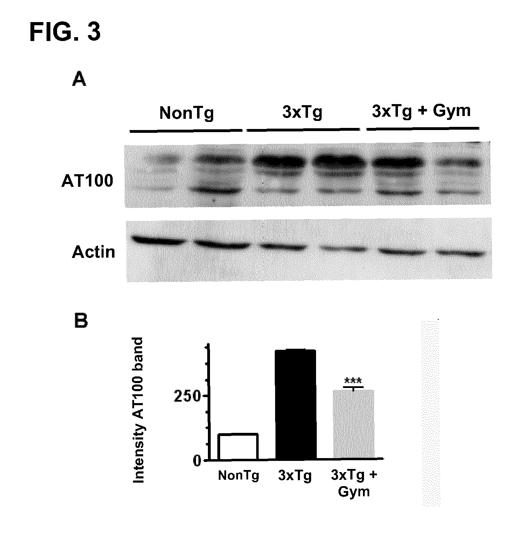

**Figure 3** shows inhibition of the expression of tau phosphorylated on residues Thr212 and Ser214 (marked with the AT100 antibody) in cultures of transgenic neurones treated with gymnodimine. (A) Western blot bands showing immunoreactivity for the AT100 antibody in wild cultures (NonTg), transgenic cultures (3xTg) and transgenic cultures treated with gymnodimine (3xTg Gym). Data obtained in a representative experiment. (B) Quantification of the expression of phosphorylated tau (marked with antibody AT100) showing a significant reduction of tau phosphorylation in transgenic cultures treated with gymnodimine (***p < 0.001, n = 3 obtained in three representative experiments, each carried out in duplicate).

### EXAMPLES

The following specific examples provided in this patent document serve to illustrate the nature of the present invention. These examples are included only for illustrative purposes and are not to be interpreted as limitations of the invention that is claimed in this document. Therefore, the examples described below illustrate the invention without limiting its field of application.

### EXAMPLE 1. DETERMINATION OF THE CELLULAR VIABILITY OF

### TREATMENT WITH GYMNODIMINE

In order to carry out the experiments of the present invention, either an *in vitro* cortical neuronal model with simultaneous overexpression of tau and β-amyloid, obtained from a model of Alzheimer's disease in triple-transgenic (3xTg-AD or 3xTg) mice, obtainable via the detailed procedure in the international patent application W02003 / 053136 and provided by the title holders of this application, or an *in vitro* cortical neuronal model obtained from non-transgenic (Non Tg) mice. The triple-transgenic neuronal model showed overexpression of presenilin (PS1_{M146V}), β-amyloid precursor protein (APP_{Swe}) and tau protein (tau_{P301L}), which gives rise to a model of Alzheimer's disease with overexpression of β-amyloid and hyperphosphorylation of tau.

The primary cortical cultures in the present invention were obtained from 3xTg-AD mouse embryos of 15-17 days gestation and the wild cultures were obtained from non-3xTg control mouse embryos of the same strain. The effect of the compounds used in this invention on cellular viability was estimated by a fluorescence assay using the Alamar Blue viability indicator. To carry out the assay, primary cortical cultures seeded on 96-well plates were used. The neuronal cells were incubated with gymnodimine, which was added to the culture medium at different concentrations, and the effect on *in vitro* neuronal viability at different treatment times was determined. The maximum concentration of gymnodimine evaluated was 100 nM and the concentration of Alamar blue was 10 %. The volume of the reaction used was 200 µl. Fluorescence was measured at wavelengths of 530 nm (excitation) and 590 nm (emission). Table 1 shows that the compounds of the present invention did not change *in vitro* cellular viability.

**Table 1: Viability of cortical neurones after treatment with different concentrations of gymnodimine for 72 hours in culture. The data are means ± standard error of three independent experiments and are expressed as percentage of control.**

| | **% control** |
|---|---|
| **Control** | 100 ± 5.9 |
| **2.5 nM Gymnodimine** | 97.0 ± 4.9 |
| **5 nM Gymnodimine** | 102.8 ± 0.3 |
| **10 nM Gymnodimine** | 105.6 ± 5.9 |
| **25 nM Gymnodimine** | 105.1 ± 1.09 |
| **50 nM Gymnodimine** | 102.9 ± 1.4 |
| **100 nM Gymnodimine** | 101.8 ± 1.7 |

### EXAMPLE 2. EFFECT OF GYMNODIMINE ON OVEREXPRESSION OF INTRACELLULAR β-AMYLOID AND TAU HYPERPHOSPHORYLATION

The effect of gymnodimine on overexpression of β-amyloid and tau hyperphosphorylation was determined by the Western blot technique. Primary neuronal cultures were treated with 50 nM gymnodimine for between 3 and 7 days of culture. The cells were then processed following the usual protocols for Western blot. For Western blot studies, protein expression was evaluated using 6E10 anti-β-amyloid primary antibodies at a dilution of 1:500, AT8 anti-Tau (tau phosphorylated on Ser202, dilution 1:1000) and AT100 anti-tau (tau phosphorylated on Thr212 and Ser214, dilution 1:1000). For Western blot assays, the neuronal cultures treated with gymnodimine were washed with cold phosphate buffer and lysed in 50 mM Tris-HCl buffer (pH 7.4) containing 150 mM NaCl, 1 mM EDTA, 1 % Triton X-100, 2 mM DTT, 2.5 mM PMSF, 40 mg / ml aprotinin, 4 mg / ml leupeptin, 5 mM NaF, 1 mM Na₃VO₄, 1 mg / ml pepstatin A and 1 mg / ml benzamidine. Total protein concentration was determined by Bradford's method using bovine albumin as standard. Aliquots of cellular lysates containing 20 µg total protein were loaded in loading buffer (50 mM Tris-HCl, 100 mM dithiothreitol, 2 % SDS, 20 % glycerol, 0.05 % bromophenol blue, pH 6.8); the proteins were separated by electrophoresis and transferred to PVDF membranes. The membranes were incubated with the primary antibodies, washed, and then incubated with a secondary antibody linked to HRP; the immunoreactivity was detected by chemiluminescence. The same membranes were reincubated with an anti-β-actin primary antibody to carry out data correction for the sample protein content. In these assays, treatment with gymnodimine was demonstrated to reduce overexpression of β-amyloid (Fig. 1) and tau phosphorylated on the Ser202 site (Fig. 2) and on the Thr212 and Ser214 (Fig 3) sites in neurones obtained from triple transgenic mice.

### EXAMPLE 3. EFFECT OF THE COMPOUNDS OF THE PRESENT INVENTION ON BASAL PHOSPHORYLATION OF TAU IN CONTROL NEURONES

The effect of gymnodimine on basal phosphorylation of tau was determined by Western blot techniques. An *in vitro* cortical neuronal model obtained from non-transgenic mice was used. Neuronal culture and sample processing was carried out in the same way as described in the previous example. The data obtained were corrected for the β-actin content of the samples. The data obtained demonstrated that treatment of control neurones with gymnodimine did not affect the basal amount of total tau (determined with the Tau46 antibody, which recognises different isoforms, both of phosphorylated tau and non-phosphorylated tau, Table 2) or the basal level of tau phosphorylated on site Ser202 (Table 3).

**Table 2: Effect of gymnodimine on total tau basal levels in control neurones. The data are means ± standard error of two independent experiments and are expressed as percentage of non-treated control.**

| | **% control** |
|---|---|
| **Control** | 100 ± 2.3 % |
| **Control + 50 nM gymnodimine** | 111 ± 3.9 % |

**Table 3: Effect of gymnodimine on the levels of tau phosphorylated on site Ser202 in control neurones. The data are expressed as percentage of non-treated control.**

| | **% control** |
|---|---|
| **Control** | 100 % |
| **Control + 50 nM gymnodimine** | 95 % |

## Claims

1. Use of a compound with the chemical structure (I) 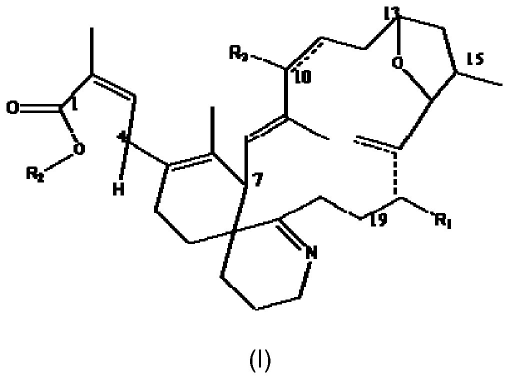 wherein:
- - - - - represents a possible double bond,
R₁ can be hydrogen, a hydroxyl (OH) or an alkoxyl (OR) group,
R₂ is hydrogen or does not exist and the oxygen binds to carbon 4 giving rise to a furanone ring,
R₃ is a hydrogen, a hydroxyl (OH) or an alkoxyl (OR) group,
for the preparation of a medicament.

2. Use of a compound according to claim 1 wherein:
there is a double bond between carbon atoms 17 and 18,
R₁ is hydrogen,
R₂ does not exist so the oxygen is bound to carbon 4 making a furanone ring, and
R₃ is a hydroxyl (OH) group.

3. Use of the compound according to any of claims 1 or 2 for the preparation of a medicament for the prevention and / or treatment of a pathology related to β-amyloid increase and / or tau hyperphosphorylation.

4. Use of a compound according to claim 3 wherein the pathology related to the β-amyloid increase is selected from the list comprising: amyotrophic lateral sclerosis, Down's syndrome, vascular dementia, cerebral amyloid angiopathy related to prion proteins and Creutzfeld-Jacob's disease.

5. Use of a compound according to claim 3 wherein the pathology related to tau hyperphosphorylation is selected from the list comprising: frontotemporal dementia, progressive supranuclear paralysis, dementia associated with multiple system tauopathy, corticobasal degeneration and frontotemporal lobular degeneration or Picks's disease.

6. Use of a compound according to claim 3 wherein the pathology related to the β-amyloid increase and tau hyperphosphorylation is selected from the list comprising: Alzheimer's disease, moderate cognitive disorders or deficits, hereditary cerebral hemorrhage with amyloidosis-Dutch type, cerebral amyloid angiopathy, dementia associated with Parkinson's disease, neurodegenerative disease due to diffuse Lewy bodies, corticobasal degeneration, sub-acute sclerosing panencephalitis, dementia with argyrophilic grain disease and familial Gerstmann-Straussler-Scheinker disease.

7. A pharmaceutical composition comprising a compound according to any of claims 1 or 2.

8. The pharmaceutical composition according to claim 7 additionally comprising a pharmaceutically acceptable vehicle.

9. The pharmaceutical composition according to either of the claims 7 or 8 additionally comprising another active ingredient.
